# EUROPÄISCHE PATENTSCHRIFT

(11) **EP 2 678 344 B1**
(45) Veröffentlichungstag und Bekanntmachung des Hinweises auf die Patenterteilung: **16.09.2015**
(21) Anmeldenummer: 12721721.4
(22) Anmeldetag: 23.02.2012
(51) Int. Cl.: C07F 9/02, B01J 31/24, C07F 9/50, C07F 15/00, C07D 311/94, C07F 1/12

(54) **CYCLOPROPENYL-YLIDEN STABILISIERTE PHOSPHENIUMKATIONEN**
CYCLOPROPENYLIDENE-STABILISED PHOSPHENIUM CATIONS
CATIONS DE PHOSPHÉNIUM STABILISÉS AU CYCLOPROPÉNYLIDÈNE

(30) Priorität: 24.02.2011 DE 102011012334
(43) Veröffentlichungstag der Anmeldung: 01.01.2014
(73) Patentinhaber: Studiengesellschaft Kohle mbH, 45470 Mülheim an der Ruhr (DE)
(72) Erfinder: ALCARAZO, Manuel, 45468 Mülheim an der Ruhr (DE); BRUNS, Hans, 47057 Duisburg (DE); PETUSKOVA, Jekaterina, 45468 Mülheim an der Ruhr (DE)
(86) Internationale Anmeldenummer: PCT/DE2012/100046
(87) Internationale Veröffentlichungsnummer: WO 2012/113393

(56) Entgegenhaltungen:
- JEKATERINA PETUSKOVA ET AL: "Cyclopropenylyliden-stabilisierte Di(aryl/alkyl)phospheniumkationen: Anwendungen in der homogenen Gold-Katalyse", ANGEWANDTE CHEMIE (INTERNATIONAL ED. IN ENGLISH), Bd. 123, Nr. 16, 14. März 2011 (2011-03-14), Seiten 3883-3886, XP55030628, ISSN: 0044-8249, DOI: 10.1002/ange.201100338
- JEKATERINA PETUSKOVA ET AL: "Synthesis, Structure, and Reactivity of Carbene-Stabilized Phosphorus(III)-Centered Trications [L 3 P] 3+", JOURNAL OF THE AMERICAN CHEMICAL SOCIETY, Bd. 133, Nr. 51, 28. Dezember 2011 (2011-12-28), Seiten 20758-20760, XP55030630, ISSN: 0002-7863, DOI: 10.1021/ja210223s
- NGOC HOA TRAN HUY ET AL: "The Phosphorus Version of the Oxaspiropentene-Cyclobutenone Rearrangement", ORGANOMETALLICS, Bd. 29, Nr. 5, 8. März 2010 (2010-03-08), Seiten 1302-1304, XP55030676, ISSN: 0276-7333, DOI: 10.1021/om900983k
- ANDREAS LANDAU, GUNTHER SEITZ: "Pseudooxokohlenstoff-Anionen der Semidreiecksäure", CHEM. BER., Bd. 124, Nr. 3, 1991, Seiten 665-669, XP002678351,
- OLIVIER BACK ET AL: "Nonmetal-Mediated Fragmentation of P 4 : Isolation of P 1 and P 2 Bis(carbene) Adducts", ANGEWANDTE CHEMIE (INTERNATIONAL ED. IN ENGLISH), Bd. 121, Nr. 30, 13. Juli 2009 (2009-07-13), Seiten 5638-5641, XP55030673, ISSN: 0044-8249, DOI: 10.1002/ange.200902344
- Ngoc Hoa Tran Huy ET AL: "Umpolung of Electrophilic Terminal Phosphinidene Complexes by Interaction with Nucleophilic Carbenes", Chemistry - An Asian Journal, vol. 4, no. 8, 3 August 2009 (2009-08-03), pages 1225-1228, XP055144776, ISSN: 1861-4728, DOI: 10.1002/asia.200900177

## Beschreibung

Die vorliegende Erfindung betrifft neue stabilisierte Cyclopropenyl-yliden-Kationen und ihre Verwendung als Liganden in Metallkatalysatoren.

Phospheniumkationen der allgemeinen Formel [R₂P:]⁺ sind isolobal mit Singulettcarbenen und können beispielsweise durch Abgabe von Elektronendichte in ihr leeres Orbital stabilisiert werden. Diese Stabilisierung kann beispielsweise durch Eingliedern des Phosphoratoms in ein heterocyclisches Gerüst A oder durch Umsetzung mit Basen unter Bildung der entsprechenden Lewis-Adukte B (Schema 1) erreicht werden.

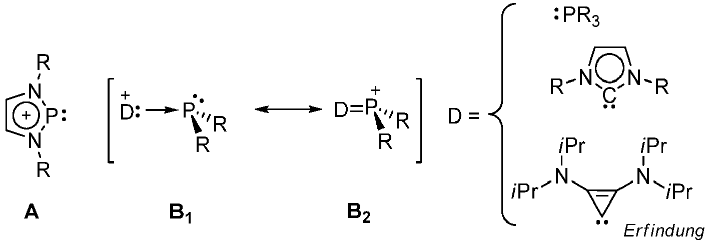

In beiden Fällen liegt die Anwesenheit des freien Elektronenpaars am Phosphoratom die Verwendung dieser Verbindungen als Liganden nahe, allerdings führt ihre intrinsische positive Ladung dazu, dass sie schwache σ-Donoren und starke π-Akzeptoren sind. Insbesondere in den Adukten vom Typ B dominiert in der Grundstruktur die Resonanzstruktur B₂, wenn D ein Phosphin ist, so dass Übergangsmetallkomplexe, die von diesen Verbindungen abgeleitet werden können, selten sind. Auch in Fällen, in denen D ein N-heterocyclisches Carben (NHC) ist, sind die Koordinationseigenschaften der resultierenden Addukte lediglich mit denen, die starke π-Akzeptor-Phosphite zeigen, vergleichbar.

Derivate der Semidreiecksaure sind grundsätzlich in Chem. Ber. 124 (1991) 665-669 beschrieben. Weiterhin erwähnt Chem. Asian J. 2009, 4, 1225 - 1228 eine Umpolung von elektrophilen endständigen Phosphiniden-Komplexen durch Wechselwirkung mit nucleophilen Carbenen.

Der vorliegenden Erfindung lag die Aufgabe zugrunde, Verbindungen zur Verfügung zu stellen, in denen die Phospheniumstruktur stabilisiert ist und die als Liganden in Edelmetallkomplexen eingesetzt werden können.

Gegenstand der vorliegenden Erfindung sind Phosphenium-Verbindungen mit der allgemeinen Formel I 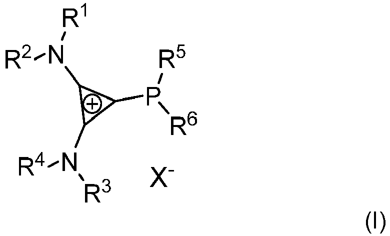 in der R¹, R², R³ und R⁴ gleich oder verschieden sind und für einen linearen oder verzweigten C₁-C₆-Alkylrest stehen, der gegebenenfalls substituiert sein kann, oder R¹ und R² und/oder R³ und R⁴ unter Bildung eines Ringes miteinander verbunden sind,
R⁵ und R⁶ für eine gesättigte oder ungesättigte, verzweigte oder lineare Alkylgruppe, Alkenylgruppe, Arylgruppe, die geeignete Substituenten, auch Heteroatome aus Substituenten, haben kann, eine Heteroatom-enthaltene Kohlenwasserstoffgruppe, die geeignete Substituenten haben kann, und die Reste R⁵ und R⁶ einen Ring bilden können, der 4- bis 20-gliedrig, gesättigt oder ungesättigt, alicyclisch oder heteroalicyclisch sein kann und geeignete Substituenten aufweisen kann, und
X- für ein Anion, ausgewählt aus BF4⁻, PF₆⁻, SbF₆⁻ und/oder BPh₄⁻, steht.

Überraschenderweise wurde festgestellt, dass die erfindungsgemäßen Phospheniumverbindungen, die mit Cyclopropenyl-yliden stabilisiert sind, in guten Ausbeuten durch Kondensation des gut verfügbaren Chlorocyclopropeniumsalzes mit sekundären Phosphinen und anschließendem Anionaustausch erhalten werden können. In einigen Fällen wird ein Anionaustausch durchgeführt, um gegebenenfalls Anionen, die aus dem Herstellungsprozess stammen, durch das entsprechende gewünschte Anion zu ersetzen. Dies ist insbesondere dann erforderlich, wenn Cl-Anionen vorliegen, die sich nachteilig auf die katalytische Aktivität auswirken können.

Die Verbindungen werden als weiße, luftstabile Feststoffe erhalten.

Bei den Verbindungen gemäß Formel I handelt es sich um sogenannte Phospheniumverbindungen. Diese Verbindungen können in zwei Grenzstrukturen dargestellt werden:

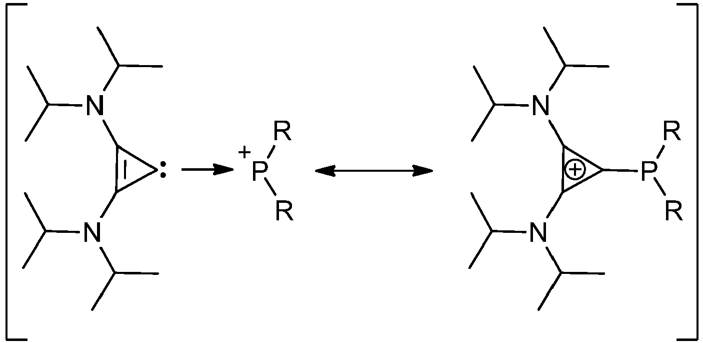

Die Erfinder haben festgestellt, dass die Stabilisierung der Kationen durch die Aminoreste am Cyclopropenium-Rest erfolgt. Besonders stabile Verbindungen werden erhalten, wenn R¹, R², R³ und R⁴ jeweils unabhängig voneinander ausgewählt sind aus iso-Propyl und tert-Butyl. Besonders bevorzugt haben R¹, R², R³ und R⁴ jeweils die gleiche Bedeutung. Besonders gut zugänglich sind Verbindungen, in denen diese Reste iso-Propyl bedeuten.

Das Anion X⁻ ist BF₄⁻, PF₆⁻, SbF₆⁻ und/oder BPh₄⁻.

Die im Rahmen der vorliegenden Erfindung verwendeten Reste R¹, R², R³ und R⁴ sind vorzugsweise ein linearer oder verzweigter C₁-C₆-Alkylrest. Die Reste R⁵ und R⁶ sind vorzugsweise ausgewählt aus linearen, oder verzweigten oder cyclischen Alkylresten, Aryl-, Alkylarylresten. Vorzugsweise stehen R⁵ und R⁶ für ggf. substituierte Phenylreste, wie C₁-C₄-Alkylphenyl, C₁-C₄-Alkoxyphenyl, Halogenophenyl, oder cyclische Alkane, wie Cyclohexyl oder Adamantyl.

Soweit der Begriff Kohlenwasserstoffgruppe verwendet wird, bedeutet er im Rahmen der Erfindung eine gesättigte oder ungesättigte, verzweigte oder lineare Alkylgruppe, Alkenylgruppe, Arylgruppe, Alkylarylgruppe, die geeignete Substituenten, auch Heteroatomsubstituenten, haben kann, oder eine Heteroatom-enthaltende Kohlenwasserstoffgruppe.

Alkyl kann unverzweigt (linear) oder verzweigt sein und hat 1 bis 6 Kohlenstoffatome. Vorzugsweise ist Alkyl Methyl, Ethyl, Propyl, Isopropyl, Butyl, Isobutyl, sec-Butyl oder tert-Butyl, ebenso Pentyl, 1-Methylpropyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. Alkyl kann auch für halogenierte Alkylreste stehen, z. B. für Trifluormethyl, Pentafluoroethyl oder 1,1,1-Trifluoroethyl.

Cycloalkyl ist vorzugsweise Cyclopropyl, Cyclobutyl, Cyclopentyl, Cyclohexyl oder Cycloheptyl, kann aber auch Adamantyl bedeuten.

Alkylen bedeutet vorzugsweise Methylen, Ethylen, Propylen, Butylen, Pentylen oder Hexylen, aber auch verzweigtes Alkylen.

Aryl ist vorzugsweise Phenyl, Naphthyl oder Diphenyl, Arylalkyl ist vorzugsweise Benzyl.

Beispiele für Substituenten sind C₁-C₄-Alkyl, wie Methyl, Ethyl, Propyl, Isopropyl, Butyl, Isobutyl, sec-Butyl oder tert-Butyl, C₁-C₄-Alkenyl, vorzugsweise Vinyl oder Propenyl, C₁C₄-Alkoxy, wie Methoxy, Ethoxy, Propoxy oder Butoxy, Aryl, Heteroaryl, Halogen, wie F, Cl, Br, I, NO₂, NH₂, usw.

Die erfindungsgemäßen Verbindungen mit der Formel I können durch Umsetzung der Halogenocyclopropeniumsalze mit Diphosphinen erhalten werden. Ein weiterer Gegenstand der vorliegenden Erfindung ist demgemäß ein Verfahren zur Herstellung von Phosphenium-Verbindungen mit der allgemeinen Formel I 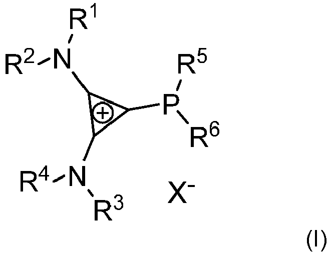 in der R¹, R², R³ und R⁴ gleich oder verschieden sind und für einen linearen oder verzweigten C₁-C₆-Alkylrest stehen, der gegebenenfalls substituiert sein kann, oder R¹ und R² und/oder R³ und R⁴ unter Bildung eines Ringes miteinander verbunden sind,
R⁵ und R⁶ für eine gesättigte oder ungesättigte, verzweigte oder lineare Alkylgruppe, Alkenylgruppe, Arylgruppe, die geeignete Substituenten, auch Heteroatome aus Substituenten, haben kann, eine Heteroatom-enthaltene Kohlenwasserstoffgruppe, die geeignete Substituenten haben kann, und die Reste R⁵ und R⁶ einen Ring bilden können, der 4- bis 20-gliedrig, gesättigt oder ungesättigt, alicyclisch oder heteroalicyclisch sein kann und geeignete Substituenten aufweisen kann,
X- für ein Anion steht,
bei dem Halogenocyclopropeniumsalze mit der allgemeinen Formel II: 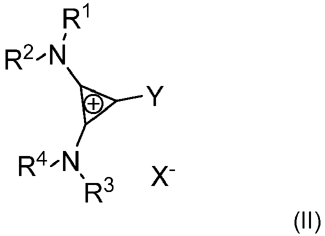 in der R¹, R², R³, R⁴ und X⁻ wie oben definiert sind und Y für ein Halogenatom steht, mit einem Phosphin der allgemeinen Formel III:

HPR⁵R⁶ III

in der R⁵ und R⁶ wie oben definiert sind, umgesetzt werden.

Die Herstellung der erfindungsgemäßen Verbindungen mit der Formel I kann auf einfache, dem Fachmann bekannte Weise erfolgen. In einer möglichen Ausführungsform des Herstellungsverfahrens werden die Ausgangsverbindungen in einem geeigneten Lösungsmittel gelöst und über einen ausreichenden Zeitraum, ggf. bei erhöhter Temperatur, bis zur Siedetemperatur des Lösungsmittels, miteinander umgesetzt. Das erhaltene Reaktionsprodukt kann ohne weitere Aufbereitung weiterverwendet werden. Die Ausgangsverbindungen reagieren nahezu quantitativ miteinander. Zur Aufbereitung des Produktes kann es in einigen Fällen erforderlich sein, das Produkt mit einem geeigneten Lösungsmittel zu waschen. Üblicherweise sind weitere Reinigungsschritte nicht erforderlich. Für den Fall, dass schädliche Anionen enthalten sind, kann zur Aufbereitung des Produktes dieses mit einer gesättigten Lösung, die die gewünschten Anionen enthält, gewaschen werden, um so einen Anionenaustausch herbeizuführen.

Als Ausgangsverbindungen mit der Formel II werden vorzugsweise solche Verbindungen eingesetzt, in denen Y für Cl steht.

Die Verbindungen mit der Formel I können in guten Ausbeuten aus dem Reaktionsgemisch isoliert werden.

Die Umsetzung der Ausgangsverbindungen zur Herstellung der erfindungsgemäßen Verbindungen mit der Formel I erfolgt vorzugsweise in einem Lösungsmittel. Als Lösungsmittel können alle üblichen polaren oder unpolaren organischen Lösungsmittel eingesetzt werden, beispielsweise CH₂Cl₂, ClCH₂CH₂C, Toluol, THF, Dimethoxyethan (DME), Dimethylformamid (DMF), Dimethylsulfoxid (DMSO), *N*-Methyl-2-pyrrolidon (NMP) oder 1,4-Dioxan und beliebige Gemische daraus.

Die erfindungsgemäßen Verbindungen mit der Formel I können als Liganden für Übergangsmetallkomplexe eingesetzt werden. Besonders geeignet sind sie als Liganden für Metallkomplexe.

Ein weiterer Gegenstand der vorliegenden Erfindung ist demgemäß die Verwendung der Verbindungen mit der Formel I als Liganden in Metallkomplexen, insbesondere in Metallkatalysatoren.

Noch ein weiterer Gegenstand der vorliegenden Erfindung sind Metallkomplexe mit der Formel IV, 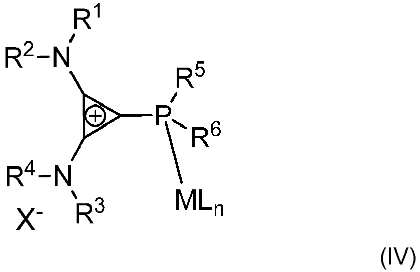 in der R¹, R², R³ und R⁴ gleich oder verschieden sind und für einen linearen oder verzweigten C₁-C₆-Alkylrest stehen, der gegebenenfalls substituiert sein kann, oder R¹ und R² und/oder R³ und R⁴ unter Bildung eines Ringes miteinander verbunden sind,
R⁵ und R⁶ für eine gesättigte oder ungesättigte, verzweigte oder lineare Alkylgruppe, Alkenylgruppe, Arylgruppe, die geeignete Substituenten, auch Heteroatome aus Substituenten, haben kann, eine Heteroatom-enthaltene Kohlenwasserstoffgruppe, die geeignete Substituenten haben kann, und die Reste R⁵ und R⁶ einen Ring bilden können, der 4- bis 20-gliedrig, gesättigt oder ungesättigt, alicyclisch oder heteroalicyclisch sein kann und geeignete Substituenten aufweisen kann,
X- für ein Anion steht,
wobei MLₙ für AuCl, AuCl₃, PdCl(allyl), RhCl(cod), RhCl(CO)₂, CuCl, RhCl₄⁻ und/oder BH₃ steht, und die Verwendung dieser Metallkomplexe als Katalysatoren.

Zur Herstellung dieser Metallkomplexe können die Verbindungen mit der Formel I mit den entsprechenden Metallverbindungen MLₙ umgesetzt werden. Die Umsetzung erfolgt üblicherweise quantitativ. Vorzugsweise wird die Reaktion der Verbindungen mit der Formel I mit den Metallverbindungen in Lösungen durchgeführt. Als Lösungsmittel sind die oben genannten Lösungsmittel geeignet.

Die zur Herstellung der Metallkomplexe eingesetzten Metallverbindungen MLₙ sind ausgewählt aus AuCl, AuCl₃, PdCl(allyl), RhCl(cod), RhCl(CO)₂, CuCl, RhCl₄⁻ und BH₃.

Die Metallkomplexe eignen sich als Katalysatoren in der organischen Synthese, beispielsweise können sie eingesetzt werden in Cycloisomerisierungen.

Beispiele für derartige Reaktionen sind nachfolgend dargestellt.

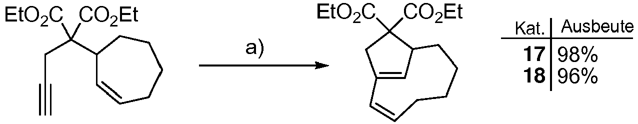

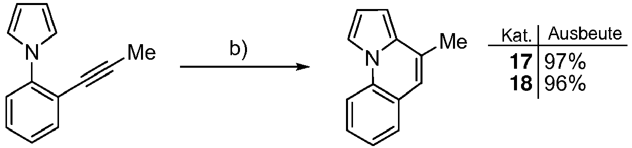

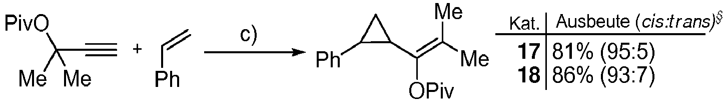

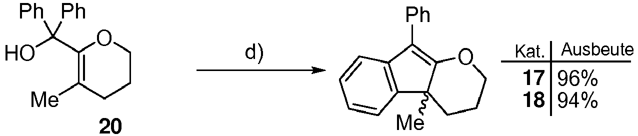

### Beispiele

Alle Reaktionen wurden in getrockneten Glaskolben unter Ar-Atmosphäre durchgeführt. Die Lösungsmittel wurden durch Destillation über Trocknungsmittel gereinigt und ebenfalls unter Argon gelagert. THF, Et₂O(Mg-Anthracen), CH₂Cl₂(HCAH₂)MeCN, Et₃N(CAH₂), MeOH(Mg), Hexan, Toluol (NA/K). Lesch-Chromatografie: Merck Silicagel 60 (230-400 Mesch), IR: Mikolet FT-7199-Spektrometer, Wellenzahlen in cm⁻¹, MS(EI): Finnigan MAT 8200 (70 eV), ISIMS: Finnigan MAT 95, Masse-Bestimmungen: Bruker APEX III FT-MS (7T Magnet), NMR: Spektrum wurden auf einem Bruker DPS 300 oder AV 400-Spektrometer in den angegebenen Lösungsmitteln aufgenommen, ¹H und ¹³H-Shift (δ sind in ppm relativ zu TMS angegeben, Kupplungskonstanten *J*) in Hz. Die Lösungsmittelsignale wurden als Referenzen verwendet und die Verschiebungen auf die TMS-Skala umgerechnet. Schmelzpunkte: Büchi-Schmelzpunktbestimmungsgerät B-540 (korrigiert). Elementaranalysen: H. Kolbe, Mülheim/Ruhr. Alle im Handel erhältlichen Verbindungen wurden, sofern es nicht anders angegeben ist, ohne weitere Aufbereitung eingesetzt. 2,3-Bis(diisopropylamino)-1-chlorocyclopropenium-Tetrafluoroborat **1** wurde gemäß Literaturverfahren hergestellt (R. Weiss, K. G. Wagner, C. Priesner, J. Macheleid, J. Am. Cem. Soc. 1985, 107, 4491-4499).

### Verbindung 2

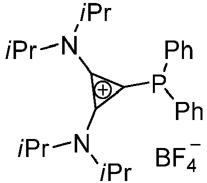

Diphenylphosphin (2,9 ml, 16,7 mmol) wurde zu einem Gemisch aus Chlorcyclopropeniumsalz **1** (2,0 g, 5,58 mmol) in THF (20 ml) zugegeben und das erhaltene Gemisch 24 Stunden auf 60 °C erwärmt. Nach Abkühlen auf Raumtemperatur wurde das Lösungsmittel im Vakuum entfernt, der Rückstand wurde in DCM (30 ml) gelöst und mit einer gesättigten Lösung von NaPF₄ (3 x 25 ml) gewaschen und über Na₂SO₄ getrocknet. Die organische Phase wurde eingeengt und der Rückstand mit Et₂O (3 x 10 ml) gewaschen. Die Verbindung wurde als weißer Feststoff (2,55 g, 90 %) erhalten.
¹H NMR (300 MHz, CD₂Cl₂) δ = 0.98 (d, *J* = 6.8 Hz, 12H), 1.29 (d, *J* = 6.8 Hz, 12H), 3.34 (sep, *J* = 6.8 Hz, 2H), 3.99 (sep, *J* = 6.8 Hz, 2H), 7.32-7.47 ppm (m, 10H). ³¹P NMR (121 MHz, CD₂Cl₂) δ = -23.61 ppm. ¹³C NMR (75 MHz, CD₂Cl₂) δ = 21.6, 22.0, 53.6, 107.1 (d, *J =* 66.8 Hz), 130.5 (d, *J =* 8.1 Hz), 131.4 (d , *J =* 8.1 Hz), 131.7, 134.7 (d, *J =* 21.2 Hz), 139.7 ppm. IR (unverdünnt) v = 695, 752, 1047, 1547, 1867, 2974 cm⁻¹. HRMS berechnet für C₂₇H₃₈N₂P⁺: 421.276711; gefunden 421.276467.

### Verbindung 3:

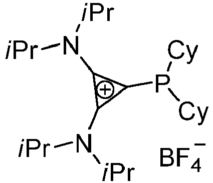

Dicyclohexylphosphin (0,84 ml, 4,17 mmol) wurde zu einem Gemisch aus Chlorcyclopropeniumsalz 1 (500 mg, 1,39 mmol) in THF (5 ml) zugefügt und das erhaltene Gemisch wurde 24 Stunden auf 60 °C erhitzt. Nach dem Abkühlen auf Raumtemperatur wurde das Lösungsmittel im Vakuum entfernt, der Rückstand in DCM (20 ml) gelöst und mit gesättigter NaBF₄-Lösung (3 x 15 ml) gewaschen und über Na₂S0₄ getrocknet. Die organische Phase wurde eingeengt und der Rückstand mit Et₂O (3 x 5 ml) gewaschen und ergab die gewünschte Verbindung als weißen Feststoff (620 mg, 86 %).
¹H NMR (300 MHz, CD₂Cl₂) δ = 0.96-1.24 (m, 8H), 1.31 (d, *J =* 6.6 Hz, 12H), 1.33 (d, *J =* 6.8 Hz, 12H), 1.35-1.51 (m, 6H), 1.55-1.95 (m, 8H), 3.94 (sep, *J =* 7.2 Hz, 2H), 4.08 (sep, *J =* 7.2 Hz, 2H). ³¹P NMR (121 MHz, CD₂Cl₂) δ = -16.77 ppm. ¹³C NMR (100 MHz, CD₂Cl₂) δ = 21.1, 21.2, 22.0 (bs), 26.5, 27.3,27.4, 27.6, 27.7, 31.5, 31.6, 31.9, 32.1, 36.2 (d, *J* = 13.7 Hz), 53.2 (bs), 107.1 (d, *J* = 78.8 Hz), 142.2 ppm. IR (unverdünnt) v = 680, 1035, 1050, 1548, 1862, 2847, 2919 cm⁻¹. (ESI)MS *berechnet für* C₂₇H₅₀N₂P⁺: 433.4; *gefunden* 433.5.

### Verbindung 4:

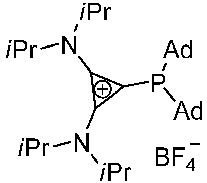

Diadamanthylphosphin (1,26 g, 4,17 mmol) wurde zu einem Gemisch aus Chlorcyclopropeniumsalz **1** (500 mg, 1,39 mmol) in THF (5 ml) zugegeben und das erhaltene Gemisch wurde 72 Stunden auf 60 °C erhitzt. Nach dem Abkühlen auf Raumtemperatur wurde das Lösungsmittel im Vakuum entfernt, der Rückstand in DCM (20 ml) gelöst und mit einer gesättigten NaPF₄-Lösung (3 x 15 ml) gewaschen und über Na₂SO₄ getrocknet. Die organische Phase wurde eingeengt und der Rückstand mit Et₂O (3 x 10 ml) gewaschen und ergab die gewünschte Verbindung als weißen Feststoff (680 mg, 79 %).
¹H NMR (300 MHz, CD₂Cl₂) δ = 1.30 (d, *J* = 6.9 Hz, 6H), 1.38 (d, *J* = 6.9 Hz, 12H), 1.45 (d, *J* = 6.9 Hz, 6H), 1.56-1.77 (m, 12H), 1.84 (bs, 12H), 2.04 (bs, 6H), 3.76-3.83 (m, 1 H), 3.92 (sep, *J* = 7.2 Hz, 1 H), 4.36 (sep, *J* = 6.9 Hz, 1 H), 4.57 (sep, *J* = 6.9 Hz, 1 H); ³¹P NMR (121 MHz, CD₂Cl₂) δ = 15.63 ppm. ¹³C NMR (75 MHz, CD₂Cl₂) δ = 20.9, 21.4, 21.5, 21.6, 23.3, 29.5 (d, *J* = 8.5 Hz), 37.0, 39.5 (d, *J* = 25.2 Hz), 42.9 (d, *J* = 11.5 Hz), 49.9, 57.5, 57.6, 105.0 (d, *J =* 90.5 Hz), 145.2 ppm (d, *J* = 22.4 Hz). IR (unverdünnt) v = 1033, 1048, 1089, 1375, 1452, 1539, 1851, 2848, 2901 cm⁻¹. HRMS *berechnet für* C₃₅H₅₈N₂P⁺: 537.433212; *gefunden* 537.433029.

### Verbindung 5:

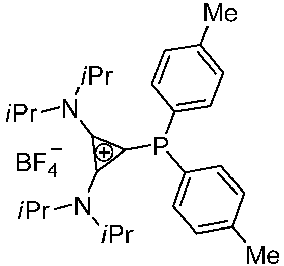

Di(p-tolyl)phosphin (1,80 g, 8,40 mmol) wurde zu einem Gemisch aus Chlorcyclopropeniumsalz **1** (1,00 g, 2,80 mmol) in THF (20 ml) gegeben und das erhaltene Gemisch wurde 24 Stunden auf 60 °C erhitzt. Nach dem Abkühlen auf Raumtemperatur wurde das Lösungsmittel im Vakuum entfernt, der Rückstand in DCM (20 ml) gelöst und mit einer gesättigten NaBF₄-Lösung (3 x 15 ml) gewaschen und über Na₂SO₄ getrocknet. Die organische Phase wurde eingeengt und der Rückstand mit Et₂O (3 x 20 ml) gewaschen, die gewünschte Verbindung wurde als weißer Feststoff erhalten (1,44 g, 96 %).
¹H-NMR (400 MHz, CD₂Cl₂) δ = 0.97 (d, *J* = 6.6 Hz, 12H), 1.27 (d, *J* = 6.8 Hz, 12H), 2.31 (s, 6H), 3.32 (sep, *J* = 6.6 Hz, 2H), 3.98 (sep, *J* = 6.8 Hz, 2H), 7.20-7.21 ppm (m, 8H). ³¹P-NMR (161 MHz, CD₂Cl₂) δ = -24.12 ppm. ¹³C NMR (121 MHz, CD₂Cl₂) δ = 21.5, 21.6, 21.9, 22.0 (bs), 53.4 (bs), 108.1 (d, *J =* 67,8 Hz), 127.9 (d, *J =* 6.1 Hz), 131.2 (d, *J =* 8.1 Hz), 134.6 (d, *J* = 22.3 Hz), 139.4, 142.4 ppm. IR (unverdünnt) v = 686, 811, 1048, 1547, 1863, 2976 cm⁻¹. HRMS *berechnet für* C₂₉H₄₂N₂P⁺: 449.308012; *gefunden* 449.308455.

### Verbindung 6:

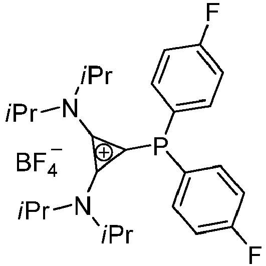

Di(p-fluoro)phosphin (2,67 g, 12,0 mmol) wurde zu einem Gemisch aus Chlorcyclopropeniumsalz 1 (1,44 g, 4,00 mmol) in THF (20 ml) zugegeben und das erhaltene Gemisch wurde 24 Stunden auf 60 °C erhitzt. Nach dem Abkühlen auf Raumtemperatur wurde das Lösungsmittel im Vakuum entfernt, der Rückstand in DCM (20 ml) gelöst und mit einer gesättigten NaBF₄-Lösung (3 x 15 ml) gewaschen und über Na₂SO₄, getrocknet. Die organische Phase wurde eingeengt und der Rückstand mit Et₂O (3 x 20 ml) gewaschen, die gewünschte Verbindung wurde als weißer Feststoff erhalten (1,65 g, 76 %).
¹H-NMR (400 MHz, CD₂Cl₂) δ = 0.99 (d, *J* = 6.8 Hz, 12H), 1.29 (d, *J* = 6.9 Hz, 12H), 3.35 (sep, *J* = 6.8 Hz, 2H), 4.00 (sep, *J* = 6.9 Hz, 2H), 7.15 (m, 4H), 7.41 ppm (m, 4H). ³¹P-NMR (161 MHz, CD₂Cl₂) δ = -24.57 ppm. ¹³C NMR (100 MHz, CD₂Cl₂) δ = 21.5, 21.6, 21.9, 54.0 (bs), 108.8 (d, *J =* 58.5 Hz), 117.8 (dd, *J =* 21.2, 9.2 Hz), 127.0 (dd, *J =* 7.1, 4.0 Hz), 137.1 (dd, *J* = 23.2, 9.1 Hz), 139.6, 165.4 ppm (d, *J* = 252.6 Hz). IR (unverdünnt) v = 691, 836, 1049, 1552, 1869, 2987 cm⁻¹. (ESI) MS *berechnet für* C₂₇H₃₆F₂N₂P⁺ 457.26, *gefunden* 457.29. HRMS *berechnet für* C₂₇H₃₆N₂O₂P⁺: 457.257868; *gefunden* 457.257700.

### Verbindung 7:

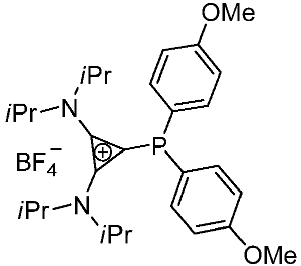

Di(p-methoxy)phosphin (0,875 g, 3,6 mmol) wurde zu einem Gemisch aus Chlorcyclopropeniumsalz **1** (430 mg, 1,2 mmol) in THF (5 ml) gegeben und das erhaltene Gemisch wurde 12 Stunden bei 60 °C erhitzt. Nach dem Abkühlen auf Raumtemperatur wurde das Lösungsmittel im Vakuum entfernt, der Rückstand in DCM (20 ml) gelöst, mit gesättigter NaBF₄-Lösung (3 x 15 ml) gewaschen und über Na₂SO₄ getrocknet Die organische Phase wurde eingeengt und der Rückstand über Silicagel-Flash-Chromatografie (DCM : Aceton = 9 : 1) gereinigt, die gewünschte Verbindung wurde als weißer Feststoff (0,55 g, 80 %) erhalten.
¹H-NMR (300 MHz, CD₂Cl₂) δ = 0.97 (d, *J* = 6.8 Hz, 12H), 1.27 (d, *J* = 6.9 Hz, 12H), 3.34 (sep, *J* = 6.8 Hz, 2H), 3.76 (s, 6H), 3.97 (sep, *J* = 6.9 Hz, 2H), 6.94 (m, 4H), 7.31 ppm (m, 4H). ³¹P-NMR (300 MHz, CD₂Cl₂) δ = -23.83 ppm. ¹³C NMR (75 MHz, CD₂Cl₂) δ = 21.6, 21.7, 22.0, 56.3, 108.1 (d, *J =* 67.4 Hz), 116.0 (d, *J =* 9.1 Hz), 122.0 (d, *J =* 3.3 Hz), 136.6 (d, *J =* 23.4 Hz), 139.2 (d, *J* = 1.3 Hz), 162.8 ppm. IR (unverdünnt) v = 684, 834, 1024, 1247, 1553, 1866, 2982 cm⁻¹; (ESI) MS *berechnet für* C₂₉H₄₂N₂O₂P⁺: 481.30 *gefunden* 481.37. HRMS *berechnet für* C₂₉H₄₂N₂O₂P⁺: 481.297843; *gefunden* 481.297370.

### Verbindung 8:

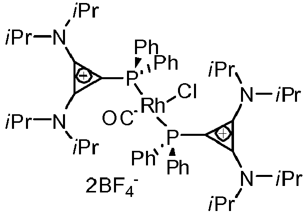

Trockenes THF (2 ml) wurde zu einem auf -20 °C gekühlten festen Gemisch aus [RhCl(CO)₂]₂ (10 mg, 0,025 mmol) und Phospheniumsalz **2** (50 mg, 0,1 mmol) gegeben. Das Reaktionsgemisch ließ man auf Raumtemperatur erwärmen und anschließend weitere 30 Minuten rühren. Nach dem Entfernen des Lösungsmittels im Vakuum wurde der feste Rückstand mit Pentan (3 x 1 ml) gewaschen und getrocknet, das gewünschte Produkt wurde als gelber Feststoff (56 mg, 93 %) erhalten.
¹H NMR (400 MHz, CD₂Cl₂) δ = 0.89 (d, *J* = 6.9 Hz, 24H), 1.27 (d, *J* = 6.9 Hz, 24H), 3.39 (m, 4H), 4.04 (m, 4H), 7.52-7.62 (m, 10H), 8.08-8.16 ppm (m, 10H). ³¹P NMR (161 MHz, CD₂Cl₂) δ = 30.15 ppm (d, *J =* 133.1 Hz). ¹³C NMR (100 MHz, CD₂Cl₂) δ = 21.2, 21.6, 55.0 (bs), 101.4 (t, *J* = 13.4 Hz), 128.5 (t, *J* = 25.8 Hz), 130.1 (t, *J* = 5.8 Hz), 133.5, 134,3 (d, *J* = 21.6 Hz), 136.1 (t, *J* = 7.8 Hz),139.2 ppm (t, *J* = 4.3 Hz). IR (unverdünnt) v = 700, 800, 1036, 1053, 1552, 1865, 1970, 2975 cm⁻¹. HRMS *berechnet für* C₅₃H₇₆BCIF₄N₄OP₂Rh⁺: 1095.427971; *gefunden* 1095.427645.

### Verbindung 9:

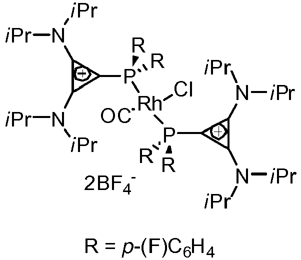

Trockenes THF (2 ml) wurde zu einem auf -20 °C gekühlten festen Gemisch aus [RhCl(CO)₂]₂ (12 mg, 0,030 mmol) und Phospheniumsalz **6** (65 mg, 0,12 mmol) gegeben. Man ließ das Reaktionsgemisch auf Raumtemperatur erwärmen und es wurde danach weitere 30 Minuten gerührt. Nach dem Entfernen des Lösungsmittels im Vakuum wurde der feste Rückstand mit Pentan (3 x 1 ml) gewaschen und getrocknet, die gewünschte Verbindung wurde als gelber Feststoff (74 mg, 99 %) erhalten.
¹H-NMR (300 MHz, CD₂Cl₂) δ = 1.02 (d, *J* = 7.0 Hz, 24H), 1.36 (d, *J* = 7.0 Hz, 24H), 3.45 (sep, *J =* 7.0 Hz, 4H), 4.13 (sep, *J =* 7.0 Hz, 4H), 7.34 (m, 8H), 8.28 ppm (m, 8H). ³¹P-NMR (300 MHz, CD₂Cl₂) δ = 26.12 ppm (d, *J =* 131.3 Hz). ¹³C NMR (75 MHz, CD₂Cl₂) δ = 21.3, 21.6, 54.0, 100.6 (t, *J =* 15.1 Hz), 117.5 (dd, *J* = 21.5, 6.3 Hz), 124.4 (t, *J =* 4.7 Hz), 138.8 (dt, *J* = 9.7, 8.5 Hz), 139.3 (dd, *J* = 7.0, 3.7 Hz), 166.0 ppm (d, *J* = 256.0 Hz). IR (unverdünnt) v = 687, 814, 1035, 1239, 1556, 1865, 1976, 2972 cm⁻¹. HRMS *berechnet für* C₅₅H₇₂B₃CIF₁₆N₄OP₂Rh⁻: 1341.399085; *gefunden* 1341.398831.

### Verbindung 10

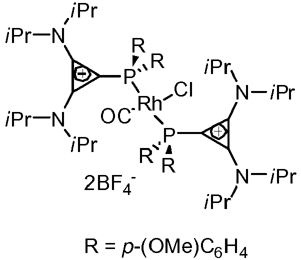

Trockenes THF (2 ml) wurden zu einem auf -20 °C gekühlten festen Gemisch aus [RhCl(CO)₂]₂ (9,0 mg, 0,023 mmol) und Phospheniumsalz 7 (52 mg, 0,091 mmol) gegeben. Man ließ das Reaktionsgemisch auf Raumtemperatur erwärmen und rührte weitere 30 Minuten. Nach dem Entfernen des Lösungsmittels im Vakuum wurde der feste Rückstand mit Pentan (3 x 1 ml) gewaschen, die gewünschte Verbindung wurde als gelber Feststoff (61 mg, 99 %) erhalten.
¹H-NMR (300 MHz, CD₂Cl₂) δ = 0.91 (d, *J* = 6.9 Hz, 24H), 1.25 (d, *J* = 6.9 Hz, 24H), 3.42 (sep, *J* = 6.9 Hz, 4H), 3.81 (s, 12H), 4.02 (sep, *J* = 6.9 Hz, 4H), 7.04 (m, 8H), 8.02 ppm (m, 8H). ³¹P-NMR (300 MHz, CD₂Cl₂) δ = 27.12 ppm (d, *J* = 130.3 Hz). ¹³C NMR (100 MHz, CD₂Cl₂) δ = 21.5, 21.9, 24.0, 56.6, 103.2 (t, *J =* 13.0 Hz), 115.8 (t, *J* = 6.7 Hz), 119.8 (t, *J =* 29.0 Hz), 138.3 (t, *J =* 8.5 Hz), 139.3, (t, *J =* 4.5 Hz), 164.2 ppm. IR (unverdünnt) v = 685, 800, 1053, 1257, 1551, 1864, 1977, 2979 cm⁻¹. HRMS *berechnet für* C₅₉H₈₄B₃ClF₁₂N₄O₅P₂Rh⁻: 1389.479175; *gefunden*: 1389.478116.

### Verbindung 11:

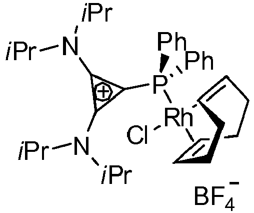

[RhCl(cod)]₂ (24,6 mg, 0,05 mmol) wurde zu einer Lösung aus **2** (50,9 mg, 0,10 mmol) in CH₂Cl₂ (2 ml) gegeben, das erhaltene Gemisch wurde eine Stunde bei Raumtemperatur gerührt. Anschließend wurde das Lösungsmittel im Vakuum entfernt und der erhaltene gelbe Rückstand mit Pentan (2 ml) gewaschen und getrocknet, **11** wurde als gelber Feststoff (72,4 mg, 96 %) erhalten.
¹H NMR (400 MHz, CD₂Cl₂) δ = 1.09 (d, *J =* 6.4 Hz, 12H), 1.33 (d, *J =* 6.8 Hz, 12H), 1.98 (m, 2H), 2.08 (m, 2H), 2.29 (m, 4H), 3.54 (bs, 2H), 3.94 (bm, 2H), 4.10 (bm, 2H), 5.58 (bs, 2H), 7.39-7.70 (m, 10H); ¹³C NMR (100 MHz, CD₂Cl₂): 21.9, 22.4, 29.5 (bs), 33.7 (d, *J =* 2.0 Hz), 53.3(bs), 59.1 (bs), 72.9 (d, *J =* 12.9 Hz), 102.9 (d, *J =* 12.9 Hz), 108.6 (dd, *J =* 7.0, 12.6 Hz), 128.0 (d, *J* = 43.9 Hz), 130.3 (d, *J =* 10.7 Hz), 133.3 (d, *J =* 1.9 Hz), 135.1 (d, *J =* 12.0 Hz), 139.2 (d, *J* = 7.9 Hz); ³¹P NMR (100 MHz, CD₂Cl₂): 25.3 (d, *J* = 150.6 Hz); IR (unverdünnt) v = 696, 752, 996, 1032, 1049, 1091, 1376, 1552, 1868, 2938, 2975 cm⁻¹; HRMS *berechnet für* C₃₅H₅₀ClN₂PRh: 667.244969; *gefunden* 667.245253.

### Verbindung 12:

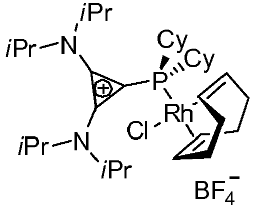

[RhCl(cod)]₂, (24,6 mg, 0,05 mmol) wurde zu einem Gemisch aus **3** (52,0 mg, 0,10 mmol) in CH₂Cl₂ (2 ml) gegeben und das erhaltene Gemisch wurde bei Raumtemperatur eine Stunde gerührt. Anschließend wurde das Lösungsmittel im Vakuum entfernt und der erhaltene gelbe Rückstand mit Pentan (2 ml) gewaschen und getrocknet, **12** wurde als gelber Feststoff (67,4 mg, 88 %) erhalten.
¹H NMR (400 MHz, CD₂Cl₂) δ = 1.23-1.45 (m, 8H), 1.50 (bs, 12H), 1.57 bs, 12H), 1.84 (bs, 4H), 1.96 (bs, 8H), 2.11-2.27 (m, 4H), 2.31-2.42 (m, 2H), 2.42-2.57 (m, 2H), 3.78 (bs, 2H), 4.22 (bs, 2H), 4.83 (bs, 2H) 5.51 (bs, 2H); ¹³C NMR (100 MHz, CD₂Cl₂): 22.2 (bs), 23.0 (bs), 26.2 (d, *J =* 1.3 Hz), 27.2, 27.3, 27.4, 27.5, 28.8 (d, *J =* 1.2 Hz), 30.5 (bs), 31.3, 31.6 (d, *J =* 2.6 Hz), 33.6 (d, *J* = 2.5 Hz), 34.6 (bs). 34.9 (bs), 58.8 (bs), 71.4 (d, *J* = 13.0 Hz), 102.2, 107.8 (dd, *J* = 12.0, 6.9 Hz), 141.4 (bs); ³¹P NMR (100 MHz, CD₂Cl₂): 28.3 (d, J = 150.5 Hz); IR (unverdünnt) v = 727, 1004, 1050, 1093, 1535, 1860, 2852, 2933 cm⁻¹; HRMS *berechnet für* C₃₅H₆₂ClN₂PRh: 679.338870; *gefunden:* 679.338987.

### Verbindung 13:

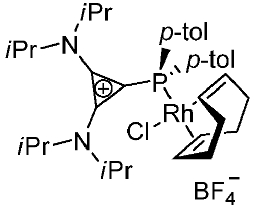

[RhCl(cod)]₂ (25,0 mg, 0,05 mmol) wurde zu einer Lösung aus **5** (54,0 mg, 0,10 mmol) in CH₂Cl₂ (2 ml) gegeben und das erhaltene Gemisch wurde eine Stunde bei Raumtemperatur gerührt. Anschließend wurde das Lösungsmittel im Vakuum entfernt und der erhaltene gelbe Feststoff mit Pentan (2 ml) gewaschen und getrocknet, **13** wurde als gelber Feststoff (77,0 mg, 99 %) erhalten.
¹H NMR (400 MHz, CD₂Cl₂) δ = 1.08 (d, *J* = 6.5 Hz, 12H), 1.32 (d, *J* = 6.7 Hz, 12H), 1.92-2.11 (m, 4H), 2.18-2.33 (m, 4H), 2.35 (s, 6H), 3.51 (m, 2H), 3.95 (m, 2H), 4.08 (m, 2H), 5.54 (m, 2H), 7.23-7.29 (m, 4H), 7.43-7.50 ppm (m, 4H). ³¹P NMR (121 MHz, CD₂Cl₂) δ = 24.66 ppm (d, *J* = 151.0 Hz). ¹³C NMR (100 MHz, CD₂Cl₂) δ = 21.9, 22.0, 22.4, 29.5, 33.7 (d, *J* = 2.9 Hz), 53.2 (bs), 56.0 (bs), 72.7 (d, *J =* 12.9 Hz), 103.5 (d, *J =* 15.7 Hz), 108.3 (dd, *J =* 12.7, 6.9 Hz), 124.5 (d, *J =* 46.0 Hz), 130.9 (d, *J =* 10.1 Hz), 135.0 (d, *J =* 12.2 Hz), 139.1 (d, *J =* 8.4 Hz), 142.2 ppm (d, *J* = 2.0 Hz). IR (neat) v = 677, 808, 1053, 1552, 1863, 2969 cm⁻¹. HRMS *berechnet für* C₃₇H₅₄ClN₂PRh⁺: 695.276269; *gefunden* 695.276236.

### Verbindung 14:

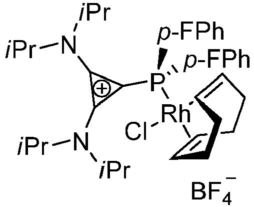

[RhCl(cod)]₂ (25,0 mg, 0,05 mmol) wurde zu einer Lösung von **6** (54,0 mg, 0,10 mmol) in CH₂Cl₂ (2 ml) gegeben und das erhaltene Gemisch wurde eine Stunde bei Raumtemperatur gerührt. Anschließend wurde das Lösungsmittel im Vakuum entfernt und der erhaltene gelbe Rückstand mit Pentan (2 ml) gewaschen und getrocknet, **14** wurde als gelber Feststoff (77,0 mg, 97 %) erhalten.
¹H NMR (300 MHz, CD₂Cl₂) δ = 1.22 (d, *J* = 6.7 Hz, 12H), 1.45 (d, *J* = 6.5 Hz, 12H), 2.05-2.28 (m, 4H), 2.30-2.56 (m, 4H), 3.65 (m, 2H), 4.03 (m, 2H), 4.21 (m, 2H), 5.70 (m, 2H), 7.26-7.37 (m, 4H), 7.73-7.86 ppm (m, 4H). ³¹P NMR (121 MHz, CD₂Cl₂) δ = 22.65 ppm (d, *J* = 153.0 Hz). ¹³C NMR (100 MHz, CD₂Cl₂) δ = 21.6, 21.9, 29.1 (bs), 33.3 (d, *J* = 2.4 Hz), 55.5 (bs), 57.6, 72.4 (d, *J* = 13.0 Hz), 101.6 (d, *J* = 19.3 Hz), 108.5 (dd, *J* = 12.4, 7.2 Hz), 117.3 (dd, *J* = 21.5, 11.5 Hz), 123.5 (dd, *J* = 45.9, 2.5 Hz), 137.4 (dd, *J* = 14.4, 8.9 Hz), 138.9 (dd, *J =* 7.2,1.4 Hz), 165.6 ppm (dd, *J* = 256.0 Hz, *J =* 2.4 Hz). IR (unverdünnt) v = 681, 815, 1053, 1548, 1865, 2976 cm⁻¹. HRMS *berechnet für* C₃₅H₄₈ClF₂N₂PRh⁺: 703.226126; *gefunden* 703.225916.

### Verbindung 15:

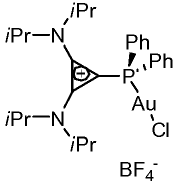

[AuCl(Me₂S)] (63,0 mg, 0,20 mmol) wurde zu einer auf -20 °C gekühlten Suspension aus Salz **2** (109,0 mg, 0,20 mmol) in trockenem THF (3 ml) zugefügt und bei dieser Temperatur eine Stunde gerührt, Das Lösungsmittel wurde im Vakuum bei 0 °C entfernt, das gewünschte Produkt wurde als ein gebrochen weißer Feststoff (154 mg, 97 %) erhalten.
¹H NMR (400 MHz, CD₂Cl₂) δ = 0.96 (d, *J* = 6.9 Hz, 12H), 1.33 (d, *J* = 6.9 Hz, 12H), 3.35 (sep, *J* = 6.9 Hz, 2H), 4.07 (sep, *J* = 6.9 Hz, 2H), 7.54-7.64 (m, 6H), 7.86-7.93 ppm (m, 4H). ³¹P NMR (161 MHz, CD₂Cl₂) δ = 17.86 ppm. ¹³C NMR (300 MHz, CD₂Cl₂) δ = 21.9, 22.0, 68.5, 96.8 (d, *J* = 45.3 Hz), 125.5 (d, *J* = 67.5 Hz), 131.1 (d, *J* = 13.6 Hz), 134.7 (d, *J* = 2.6 Hz), 135.9 (d, *J* = 16.2 Hz), 138.7 ppm. IR (unverdünnt) v = 692, 751, 1057, 1568, 1866, 2979 cm⁻¹. HRMS *berechnet für* C₂₇H₃₈AuClN₂P⁺: 653.212121; *gefunden* 653.213006.

### Verbindung 16:

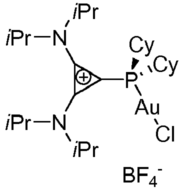

[AuCl(Me₂S)] (29,5 mg, 0,10 mmol) wurden zu einer auf -20 °C gekühlten Suspension von Salz **3** (52,0 mg, 0,10 mmol) in trockenem CH₂Cl₂ (3 ml) gegeben und bei dieser Temperatur eine Stunde gerührt. Das Lösungsmittel wurde im Vakuum bei 0 °C entfernt, das gewünschte Produkt wurde als ein gebrochen weißer Feststoff (70,8 mg, 94 %) erhalten.
¹H NMR (300 MHz, CD₂Cl₂) δ = 1.23-1.45 (m, 10H), 1.51 (d, *J* = 5.7 Hz, 12H), 1.63 (d, *J* = 6.9 Hz, 12H), 1.65-1.82 (m, 4H), 1.94 (bs, 4H), 2.10-2.16 (m, 2H), 2.36-2.50 (m, 2H), 4.16 (sep, *J* = 6.9 Hz, 2H), 4.27 ppm (sep, *J* = 6.9 Hz, 2H). ³¹P NMR (121 MHz, CD₂Cl₂) δ = 33.31 ppm. ¹³C NMR (75 MHz, CD₂Cl₂) δ = 22.0, 22.1, 25.8 (bs), 26.8 (d, *J* = 15.5 Hz), 27.1 (d, *J* = 13.4 Hz), 30.8, 30.9, 32.1, 32.2, 38.0 (d, *J* = 32.1 Hz), 54.5 (bs), 68.5, 95.1 (bs), 141.1 ppm. IR (unverdünnt) v = 682, 1029, 1051, 1558, 1863, 2851, 2928 cm⁻¹. HRMS *berechnet für* C₂₇H₅₀AuClN₂P⁺: 665.306018; *gefunden* 665.306796.

### Verbindung 17:

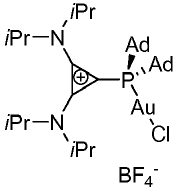

[AuCl(Me₂S)] (29,5 mg, 0,10 mmol) wurde zu einer auf -20 °C gekühlten Suspension des Salzes **4** (62,5 mg, 0,10 mmol) in trockenem CH₂Cl₂ (3 ml) gegeben und bei dieser Temperatur eine Stunde gerührt. Das Lösungsmittel wurde dann im Vakuum bei 0 °C entfernt, das gewünschte Produkt wurde als ein gebrochen weißer Feststoff (81,4 mg, 95 %) erhalten.
¹H NMR (400 MHz, CD₂Cl₂) δ = 1.28 (d, *J* = 6.4 Hz, 8H), 1.39 (d, *J* = 6.8 Hz, 12H), 1.62 (bs, 6H), 1.71 (s, 10H), 2.00-2.14 (m, 18H), 4.12 (bs, 1 H), 4.36 (bs, 1 H), 4.43 ppm (sep, *J* = 6.8 Hz, 2H). ³¹P NMR (161 MHz, CD₂Cl₂) δ = 33.31 ppm (bs). ¹³C NMR (100 MHz, CD₂Cl₂) δ = 21.7 (bs), 22.7 (bs), 23.0, 28.9, 29.1, 36.0, 36.1, 42.3, 42.4, 44.7 (bs), 54.0, 54.2, 54.3, 58.2, 93.7 (bs), die beiden C-N aus den Cyclopropeniumkationen wurden nach verlängerter Messung nicht nachgewiesen IR (unverdünnt) v = 672, 734, 1055, 1551, 1842, 2899 cm⁻¹. HRMS *berechnet für* C₃₅H₅₈AuClN₂P⁺: 769.368616; *gefunden* 769.368204.

### Verbindung 18:

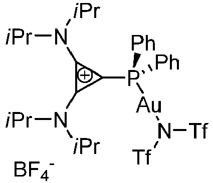

AgNTf₂ (77,6 mg, 0,20 mmol) wurde zu einer auf 0 °C gekühlten Suspension des Komplexes **15** (148,2 mg, 0,20 mmol) in trockenem CH₂Cl₂ (3 ml) zugefügt und bei dieser Temperatur eine Stunde gerührt. Das Reaktionsgemisch wurde durch einen Celite Plug filtriert. Nach Entfernen des Lösungsmittels im Vakuum wurde das gewünschte Produkt als ein gebrochen weißer Feststoff (169 mg, 96 %) erhalten.
¹H NMR (400 MHz, CD₂Cl₂) δ = 0.95 (d, *J* = 7.2 Hz, 12H), 1.32 (d, *J* = 7.2 Hz, 12H), 3.27 (sep, *J* = 6.8 Hz, 2H), 4.08 (sep, *J* = 6.8 Hz, 2H), 7.52-7.66 (m, 6H), 7.81-7.95 ppm (m, 4H). ³¹P NMR (161 MHz, CD₂Cl₂) δ = 14.47 ppm. ¹³C NMR (400 MHz, CD₂Cl₂) δ = 21.9, 22.0, 55.4 (bs), 94.6 (d, *J* = 54.8 Hz), 120.2 (q, *J* = 320.2 Hz), 124.3 (d, *J* = 71.6 Hz), 131.4 (d, *J* = 13.5 Hz), 135.2 (d, *J* = 2.5 Hz), 135.9 (d, 16.8 Hz), 139.0 ppm (d, *J* = 7.6 Hz). IR (unverdünnt) v = 691, 956, 1052, 1130, 1182, 1351, 1401, 1439, 1567, 1866, 2986 cm⁻¹. HRMS *berechnet für* C₂₉H₃₈AuClN₃O₄F₆PS₂⁺: 898.160568; *gefunden* 898.160583.

### Verbindung 19:

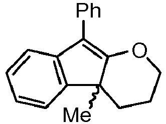

Unter Rühren wurde zu einer Lösung des Alkohols 20 (s. Seite 7) (0,1 mmol) in trockenem DCM (2 ml) des Katalysators 17 (1,0 mg, 0,001 mmol) gegeben und das Reaktionsgemisch bei Raumtemperatur 30 Minuten gerührt. Das Reaktionsgemisch wurde über ein "Silica Plug " abfiltriert. Nach Entfernen des Lösungsmittels im Vakuum wurde die Zielverbindung (27 mg, 97 % Ausbeute) erhalten.
¹H NMR (300 MHz, CD₂Cl₂) δ = 1.30 (s, 3H), 1.45-16.1 (m, 2H), 2.13-2.31 (m, 2H), 3.54-3.64 (m, 1H), 4.28-4.36 (m, 1H), 7.06 (dt, *J* = 7.3, 1.3 Hz, 1H), 7.14 (dt, *J* = 7.4, 1.4 Hz, 1H), 7.18-7.26 (m, 2H), 7.31-7.38 (m, 3H), 7.47-7.52 ppm (m, 2H). ¹³C NMR (300 MHz, CD₂Cl₂) δ = 22.9, 23.0, 33.8, 45.2, 73.3, 118.2, 120.3, 121.8, 124.6, 127.3, 127.5, 129.0, 129.4, 133.9, 142.3, 149.7, 166.2 ppm. IR (unverdünnt) v = 757, 1002, 1069, 1140, 1305, 1445, 1619, 2874, 2931 cm⁻¹. HRMS *berechnet für* C₁₉H₁₈O: 262.135762; *gefunden*: 262.135495.

### Verbindung 20:

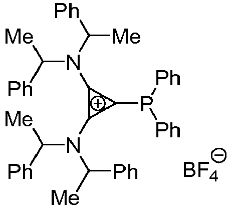

Diphenylphosphin (1,3 ml, 7,6 mmol) wurde zu einem Gemisch aus dem entsprechenden Chlorcyclopropeniumsalz (1,56 g, 2,5 mmol) in THF (10 ml) zugegeben und das erhaltene Gemisch 24 Stunden auf 60 °C erwärmt. Nach Abkühlen auf Raumtemperatur wurde das Lösungsmittel im Vakuum entfernt, der Rückstand wurde in DCM (20 ml) gelöst und mit einer gesättigten Lösung von NaBF₄ (3 x 25 ml) gewaschen und über Na₂SO₄ getrocknet. Die organische Phase wurde eingeengt und der Rückstand mit Et₂O (3 x 10 ml) gewaschen. Die Verbindung wurde als weißer Feststoff (0,96 g, 48 %) erhalten.
¹H NMR (300 MHz, CDCl₃) δ = 1.44 (d, *J* = 7.2 Hz, 6H), 1.83 (d, *J* = 7.2 Hz, 6H), 4.26 (q, *J =* 7.2 Hz, 2H), 4.62 (q, *J* = 7.2 Hz, 2H), 6.48-6.57 (m, 4H), 6.72-6.81 (m, 4H), 6.95-7.09 (m, 12H), 7.22-7.39 (m, 8H),7.41-7.49 ppm (m, 2H). ³¹P NMR (121 MHz, CDCl₃) δ = -22.31 ppm. ¹³C NMR (75 MHz, CDCl₃) δ = 17.6, 18.0, 57.3 (br.s), 60.2 (br.s) 109.3 (d, *J =* 69.4 Hz), 126.2 (d, *J* = 27.2 Hz), 127.5, 127.7 (d, *J* = 17.5 Hz), 128.3 (d, *J* = 7.9 Hz), 128.6 (d, *J* = 7.8 Hz), 129.5 (d, *J =* 11.0 Hz), 130.7 (d, *J =* 10.1 Hz), 132.7, 133.0, 133.2, 135.7, 136.4, 140.3 ppm (d, *J* = 2.0 Hz),. IR (unverdünnt) v =694, 1050, 1524, 1858, 3054 cm⁻¹. HRMS berechnet für C₄₇H₄₆N₂P⁺: 669.339313; gefunden 669.339366.

### Verbindung 21:

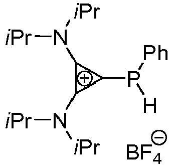

Phenylphosphin (7.4 mL, 5.3 mmol, 10 % Lösung in Hexane) wurde zu einem Gemisch aus Chlorcyclopropeniumsalz 1 (630.0 mg, 1.8 mmol) in THF (6 ml) zugegeben und das erhaltene Gemisch 24 Stunden auf 60 °C erwärmt. Nach Abkühlen auf Raumtemperatur wurde das Lösungsmittel im Vakuum entfernt, der Rückstand wurde in DCM (15 ml) gelöst und mit einer gesättigten Lösung von NaBF₄ (3 x 25 ml) gewaschen und über Na₂SO₄ getrocknet. Die Verbindung wurde als weißer Feststoff (575 mg, 76 %).
¹H NMR (300 MHz, CDCl₃) δ = 1.12 (d, *J* = 6.9 Hz, 6H), 1.31-1.46 (m, 18H), 3.64-3.80 (m, 2H), 4.14 (sept, *J* = 6.9 Hz, 2H), 5.63 (d, *J* = 233.6 Hz, 1 H) 7.44-7.51 (m, 3H), 7.64-7.72 ppm (m, 2H). ³¹P NMR (161 MHz, CDCl₃) δ = -70.88 ppm. ¹³C NMR (100 MHz, CD₂Cl₂) δ = 21.6, 21.8, 22.9, 50.8, 56.7, 105.5 (d, *J* = 58.7 Hz), 126.0, 130.4 (d, *J* = 8.1 Hz), 132.3, 137.1 (d, *J* = 20.2 Hz), 138.7 ppm (d, *J* = 4.1 Hz). IR (unverdünnt) v = 729, 1032, 1349, 1558, 1872, 2984 cm⁻¹. HRMS berechnet für C₂₁H₃₄N₂P⁺: 345.245415; gefunden 345.245568.

### Verbindung 22:

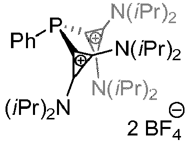

KHMDS (215.7 mg, 1.08 mmol) wurde zu einem Gemisch aus Salz 21(425.0 mg, 0.98 mmol) in THF (6 ml) zugegeben und das erhaltene Gemisch 2 Stunden bei -40 °C gerührt. Das Lösungsmittel wurde im Vakuum entfernt, der Rückstand wurde in DCM (15 ml) gelöst und mit einer gesättigten Lösung von NaBF₄ (3 x 15 ml) gewaschen und über Na₂SO₄ getrocknet. Die organische Phase wurde eingeengt und der Rückstand mit THF (3 x 10 ml) gewaschen. Die Verbindung wurde als weißer Feststoff (0,5156 g, 69 %) erhalten.
¹H NMR (400 MHz, CD₂Cl₂) δ = 1.21 (d, *J =* 6.9 Hz, 12H), 1.25 (d, *J =* 6.9 Hz, 12H), 1.38 (d, *J* = 6.9 Hz, 12H), 1.44 (d, *J =* 6.9 Hz, 12H), 3.64 (sept, *J* = 6.9 Hz, 4H), 4.17 (sept, *J* = 6.9 Hz, 4H), 7.62-7.68 (m, 3H), 7.73-7.80 ppm (m, 2H). ³¹P NMR (161 MHz, CD₂Cl₂) δ = -48.31 ppm. ¹³C NMR (100 MHz, CD₂Cl₂) δ = 21.2, 21.5, 21.5, 21.6, 21.6, 21.7, 53.4, 54.8, 98.2 (d, *J* = 59.5 Hz), 125.0 (d, *J =* 4.6 Hz), 131.1 (d, *J =* 8.9 Hz), 133.0, 134.8 (d, *J =* 22.9 Hz), 140.0 ppm. IR (unverdünnt) v = 694, 1029, 1151, 1357, 1555, 1858, 2974 cm⁻¹. HRMS berechnet für C₃₆H₆₁BF₄N₄P⁺: 667.467509; gefunden 667.467401.

### Verbindung 23:

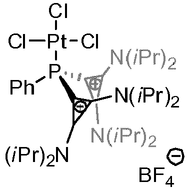

K₂PtCl₄ (44,8 mg, 0,117 mmol) wurde zu einem Gemisch aus Salz **35** (88 mg, 0.117 mmol) in CH₃CN (2 ml) zugegeben und das erhaltene Gemisch 16 Stunden gerührt. Nach Abkühlen auf Raumtemperatur wurde das Lösungsmittel im Vakuum entfernt und der Rückstand wurde in DCM (5 ml) gelöst, filtriert und die Lösungsmittel verdampfen lassen. Die Verbindung wurde als gelber Feststoff (111 mg, 98 %) erhalten.
¹H NMR (400 MHz, CD₂Cl₂) δ = 1.10 (d, *J =* 6.4 Hz, 12H), 1.14 (d, *J* = 6.4 Hz, 12H), 1.33 (d, *J* = 7.0 Hz, 12H), 1.36 (d, *J* = 7.0 Hz, 12H), 4.05-4.18 (m, 4H), 4.29-4.43 (m, 4H), 7.58-7.67 (m, 3H), 8.37-8.46 ppm (m, 2H).³¹P NMR (161 MHz, CD₂Cl₂) δ = -21.24 ppm (*J* = 1994.8 Hz). ¹³C NMR (100 MHz, CD₂Cl₂) δ = 17.5, 18.1, 18.5, 53.4, 90.6 (d, *J* = 51.5 Hz), 118.9 (d, *J* = 70.8 Hz), 126.8 (d, *J =* 12.9 Hz), 130.9 (d, *J =* 2.3 Hz), 133.5 (d, *J =* 13.5 Hz), 134.7 ppm (d, *J* = 8.6 Hz). IR (unverdünnt) v = 679, 1050, 1148, 1376, 1557, 1850, 2977 cm⁻¹. HRMS berechnet für C₃₆H₆₁Cl₃N₄PPt⁺: 880.333066; gefunden 880.333903.

Unter Verwendung von Verbindung 23 wurde 2-Ethinyl-1,1'-Binaphthyl in Gegenwart von Ag(C₂B₁₀H₅Cl₆) zu 95% zu [5]Helicene umgesetzt.

## Patentansprüche

1. Phosphenium-Verbindungen mit der allgemeinen Formel I: 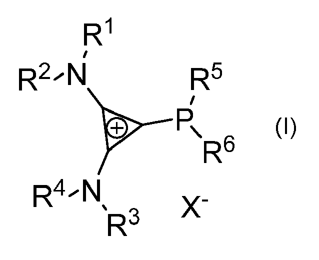 in der R¹, R², R³ und R⁴ gleich oder verschieden sind und für einen linearen oder verzweigten C₁-C₆-Alkylrest stehen, der gegebenenfalls substituiert sein kann, oder R¹ und R² und/oder R³ und R⁴ unter Bildung eines Ringes miteinander verbunden sind,
R⁵ und R⁶ für eine gesättigte oder ungesättigte, verzweigte oder lineare Alkylgruppe, Alkenylgruppe, Arylgruppe, die geeignete Substituenten, auch Heteroatome aus Substituenten, haben kann, eine Heteroatom-enthaltene Kohlenwasserstoffgruppe, die geeignete Substituenten haben kann, und die Reste R⁵ und R⁶ einen Ring bilden können, der 4- bis 20-gliedrig, gesättigt oder ungesättigt, alicyclisch oder heteroalicyclisch sein kann und geeignete Substituenten aufweisen kann, und
X- für ein Anion, ausgewählt aus BF₄⁻, PF₆⁻, SbF₆⁻ und/oder BPh₄⁻, steht.

2. Phosphenium-Verbindungen nach Anspruch 1, **dadurch gekennzeichnet, dass** R¹, R², R³ und R⁴ jeweils unabhängig voneinander ausgewählt sind aus iso-Propyl und tert-Butyl.

3. Verfahren zur Herstellung von Phosphenium-Verbindungen mit der allgemeinen Formel I nach Anspruch 1 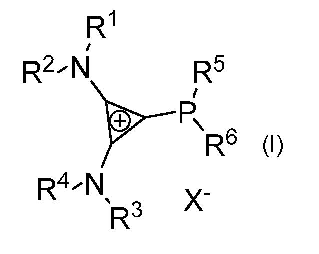 in der R¹, R², R³ und R⁴ gleich oder verschieden sind und für einen linearen oder verzweigten C₁-C₆-Alkylrest stehen, der gegebenenfalls substituiert sein kann, oder R¹ und R² und/oder R³ und R⁴ unter Bildung eines Ringes miteinander verbunden sind,
R⁵ und R⁶ für eine gesättigte oder ungesättigte, verzweigte oder lineare Alkylgruppe, Alkenylgruppe, Arylgruppe, die geeignete Substituenten, auch Heteroatome aus Substituenten, haben kann, eine Heteroatom-enthaltene Kohlenwasserstoffgruppe, die geeignete Substituenten haben kann, und die Reste R⁵ und R⁶ einen Ring bilden können, der 4- bis 20-gliedrig, gesättigt oder ungesättigt, alicyclisch oder heteroalicyclisch sein kann und geeignete Substituenten aufweisen kann,
X- für ein Anion steht,
bei dem Halogenocyclopropeniumsalze mit der allgemeinen Formel II: 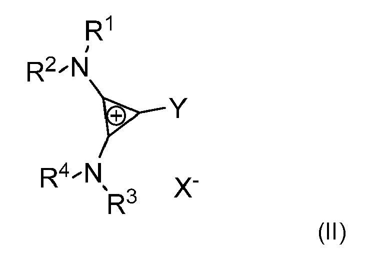 in der R¹, R², R³, R⁴ und X⁻ wie oben definiert sind und Y für ein Halogenatom steht, mit einem Phosphin der allgemeinen Formel III:
HPR⁵R⁶ III
in der R⁵ und R⁶ wie oben definiert sind,
umgesetzt werden.

4. Verfahren nach Anspruch 3, **dadurch gekennzeichnet, dass** X für Chlor steht.

5. Verwendung der Verbindungen mit der Formel I als Liganden in Metallkomplexen.

6. Metallkomplexe mit der allgemeinen Formel IV: 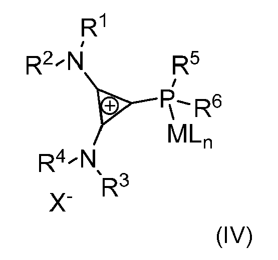 in der R¹, R², R³ und R⁴ gleich oder verschieden sind und für einen linearen oder verzweigten C₁-C₆-Alkylrest stehen, der gegebenenfalls substituiert sein kann, oder R¹ und R² und/oder R³ und R⁴ unter Bildung eines Ringes miteinander verbunden sind, R⁵ und R⁶ für eine gesättigte oder ungesättigte, verzweigte oder lineare Alkylgruppe, Alkenylgruppe, Arylgruppe, die geeignete Substituenten, auch Heteroatome aus Substituenten, haben kann, eine Heteroatom-enthaltene Kohlenwasserstoffgruppe, die geeignete Substituenten haben kann, und die Reste R⁵ und R⁶ einen Ring bilden können, der 4- bis 20-gliedrig, gesättigt oder ungesättigt, alicyclisch oder heteroalicyclisch sein kann und geeignete Substituenten aufweisen kann,
X- für ein Anion steht,
wobei MLₙ für AuCl, AuCl₃, PdCl(allyl), RhCl(cod), RhCl(CO)₂, CuCl, RhCl₄⁻ und/oder BH₃ steht.

7. Verwendung der Verbindungen nach Anspruch 6 als Katalysatoren in der organischen Synthese in Cycloisomerisierungen.

## Claims

1. Phosphenium compounds having the general formula I: 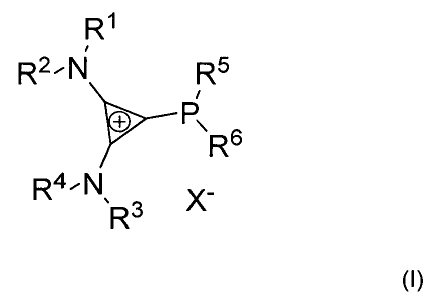 in which R¹, R², R³ and R⁴ are identical or different and represent a linear or branched C₁-C₆-alkyl radical, which can optionally be substituted, or R¹ and R² and/or R³ and R⁴ are bonded to one another with the formation of a ring,
R⁵ and R⁶ stand for a saturated or unsaturated, linear or branched alkyl group, alkenyl group or aryl group, which can have suitable substituents, even heteroatoms of substituents, or a heteroatom-comprising hydrocarbon group, which can have suitable substituents, and the R⁵ and R⁶ radicals can form a ring which can be 4- to 20-membered, saturated or unsaturated, alicyclic or heteroalicyclic and can have suitable substituents,
X- represents an anion selected from BF₄⁻, PF₆⁻, SbF₆⁻ and/or BPh₄⁻.

2. Phosphenium compounds as claimed in claim 1, **characterized in that** R¹, R², R³ and R⁴, each independently of one another, are chosen from isopropyl and tert-butyl.

3. A process for the preparation of phosphenium compounds with the general formula I according to claim1: 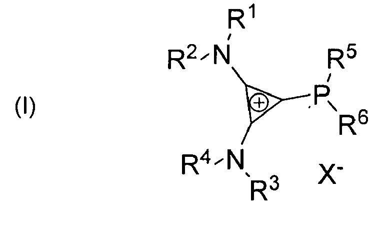 in which R¹, R², R³ and R⁴ are identical or different and represent a linear or branched C₁-C₆-alkyl radical, which can optionally be substituted, or R¹ and R² and/or R³ and R⁴ are bonded to one another with the formation of a ring,
R⁵ and R⁶ stand for a saturated or unsaturated and linear or branched alkyl group, alkenyl group or aryl group, which can have suitable substituents, even heteroatoms of substituents, or a heteroatom-comprising hydrocarbon group, which can have suitable substituents, and the R⁵ and R⁶ radicals can form a ring which can be 4- to 20-membered, saturated or unsaturated, alicyclic or heteroalicyclic and can have suitable substituents,
X- represents an anion,
in which halocyclopropenium salts having the general formula II: 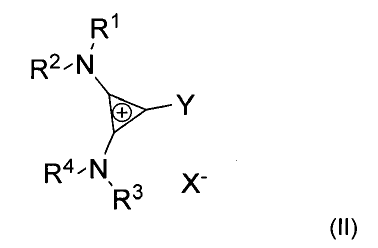 in which R¹, R², R³ and R⁴ are defined as above and Y represents a halogen atom, are reacted with a phosphine of the general formula III:
HPR⁵R⁶ III
in which R⁵ and R⁶ are defined as above.

4. Process as claimed in claim 3, **characterized in that** X represents chlorine.

5. Use of the compounds having the formula I as ligands in metal complexes.

6. Metal complexes with the general formula IV: 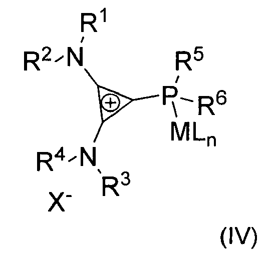 in which R¹, R², R³ and R⁴ are identical or different and represent a linear or branched C₁-C₆-alkyl radical, which can optionally be substituted, or R¹ and R² and/or R³ and R⁴ are bonded to one another with the formation of a ring,
R⁵ and R⁶ stand for a saturated or unsaturated, linear or branched alkyl group, alkenyl group or aryl group, which can have suitable substituents, even heteroatoms as substituents, or a heteroatom-comprising hydrocarbon group, which can have suitable substituents, or the R⁵ and R⁶ radicals can form a ring which can be 4- to 20-membered, saturated or unsaturated and alicyclic or heteroalicyclic and can have suitable substituents,
X⁻ represents an anion,
MLₙ represents AuCl, AuCl₃, PdCl(allyl), RhCl(cod), RhCl(CO)₂, CuCl, RhCl₄⁻ and/or BH₃.

7. Use of the compounds as claimed in claim 6 as catalysts in organic synthesis in cycloisomerizations.

## Revendications

1. Composés de phosphénium de la formule générale I : 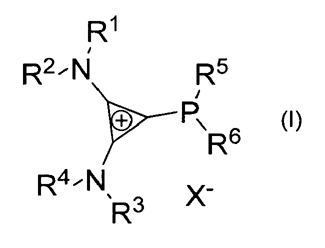 dans laquelle R¹, R², R³ et R⁴ sont identiques ou différents et représentent un reste alkyle en C₁ à C₆, linéaire ou ramifié qui peut le cas échéant, être substitué, ou bien R¹ et R² et/ou R³ et R⁴ sont reliés entre eux en formant un noyau cyclique,
R⁵ et R⁶ représentent un groupe alkyle, alcényle, aryle, ramifié ou linéaire, saturé ou insaturé, qui peut porter des substituants adéquats, y compris des hétéroatomes de substituants, un groupe hydrocarbure contenant un hétéroatome, pouvant porter des substituants adéquats et les restes R⁵ et R⁶ peuvent former un noyau cyclique, de 4 à 20 termes, qui peut être saturé ou insaturé, alicyclique ou hétéro-alicyclique et qui peut porter des substituants adéquats et
X- représente un anion, choisi parmi BF₄⁻, PF₆⁻, SbF₆⁻ et/ou BPh₄⁻.

2. Composés de phosphénium selon la revendication 1, **caractérisé en ce que** chaque fois indépendamment l'un de l'autre, R¹, R², R³ et R⁴ sont choisis parmi l'isopropyle et le tert-butyle.

3. Procédé destiné à préparer des composés de phosphénium, à l'aide de la formule générale I selon la revendication 1 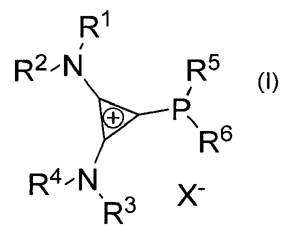 dans laquelle R¹, R², R³ et R⁴ sont identiques ou différents et représentent un reste alkyle en C₁ à C₆, linéaire ou ramifié qui peut le cas échéant, être substitué, ou bien R¹ et R² et/ou R³ et R⁴ sont reliés entre eux en formant un noyau cyclique,
R⁵ et R⁶ représentent un groupe alkyle, alcényle, aryle, ramifié ou linéaire, saturé ou insaturé, qui peut porter des substituants adéquats, y compris des hétéroatomes de substituants, un groupe hydrocarbure contenant un hétéroatome, pouvant porter des substituants adéquats et les restes R⁵ et R⁶ peuvent former un noyau cyclique, de 4 à 20 termes, qui peut être saturé ou insaturé, alicyclique ou hétéro-alicyclique et qui peut porter des substituants adéquats et
X- représente un anion,
lors duquel on transforme des sels d'halogéno-cyclopropénium de la formule générale II : 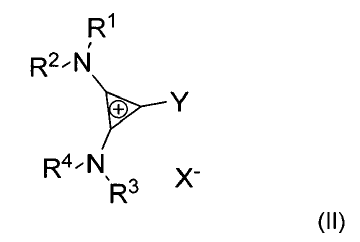 dans laquelle R¹, R², R³, R⁴ et X⁻ sont définis tel que ci-dessus et Y représente un atome d'halogène,
avec une phosphine de la formule générale III :
HPR⁵R⁶ III
dans laquelle R⁵ et R⁶ sont définis tels que ci-dessus.

4. Procédé selon la revendication 3, **caractérisé en ce que** X représente du chlore.

5. Utilisation des composés de la formule I en tant que ligands dans des complexes métalliques.

6. Complexes métalliques de la formule générale IV : 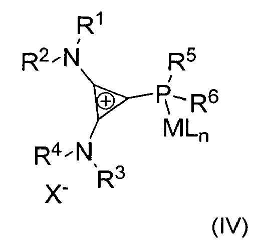 dans laquelle R¹, R², R³ et R⁴ sont identiques ou différents et représentent un reste alkyle en C₁ à C₆, linéaire ou ramifié qui peut le cas échéant, être substitué, ou bien R¹ et R² et/ou R³ et R⁴ sont reliés entre eux en formant un noyau cyclique,
R⁵ et R⁶ représentent un groupe alkyle, alcényle, aryle, ramifié ou linéaire, saturé ou insaturé, qui peut porter des substituants adéquats, y compris des hétéroatomes de substituants, un groupe hydrocarbure contenant un hétéroatome, pouvant porter des substituants adéquats et les restes R⁵ et R⁶ peuvent former un noyau cyclique, de 4 à 20 termes, qui peut être saturé ou insaturé, alicyclique ou hétéro-alicyclique et qui peut porter des substituants adéquats et
X- représente un anion,
MLₙ représentant l'AuCl, l'AuCl₃, le PdCI(allyle), le RhCl(cod), le RhCl(CO)₂, le CuCl, le RhCl₄⁻ et/ou le BH₃.

7. Utilisation des composés selon la revendication 6 en tant que catalyseurs dans la synthèse organique lors de cycloisomérisations.
